(19) Europäisches Patentamt
European Patent Office
Office européen des brevets

(11) Publication number : 0 518 807 A1

# (12) EUROPEAN PATENT APPLICATION

(21) Application number : **92810258.1**

(22) Date of filing : **03.04.92**

(51) Int. Cl.[5] : **C07D 211/46,** C07D 211/94, C07D 493/10, // C08K5/3435 , (C07D493/10, 319:00, 319:00)

(30) Priority : **12.04.91 IT MI911019**

(43) Date of publication of application : **16.12.92 Bulletin 92/51**

(84) Designated Contracting States : **BE DE ES FR GB IT NL**

(71) Applicant : **CIBA-GEIGY AG**
**Klybeckstrasse 141**
**CH-4002 Basel (CH)**
(84) **BE DE ES FR GB NL**

(71) Applicant : **CIBA-GEIGY S.p.A.**
**Strada Statale 233-Km. 20,5**
**Origgio (IT)**
(84) **IT**

(72) Inventor : **Scrima, Roberto, Dr.**
**Via del Borgo di S. Pietro 132**
**I-40126 Bologna (IT)**
Inventor : **Zagnoni, Graziano**
**via Fornaci, 77**
**I-40038 Vergato-Bologna (IT)**
Inventor : **Borzatta, Valerio, Dr.**
**Via Novelli 2**
**Bologna (IT)**

(54) **Piperidine compounds for use as stabilisers for organic materials.**

(57) The present invention relates to novel piperidine compounds of the formula (I)

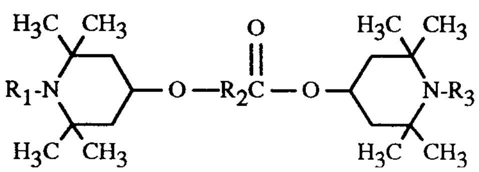

(I)

in which $R_1$ and $R_3$ which are identical or different are hydrogen, $C_1$-$C_8$alkyl, O, OH, NO, $CH_2CN$, $C_1$-$C_{18}$alkoxy, $C_5$-$C_{12}$cycloalkoxy, $C_3$-$C_6$alkenyl, $C_7$-$C_9$phenylalkyl which is unsubstituted or mono-, di- or tri-substituted on the phenyl by $C_1$-$C_4$alkyl ; or $C_1$-$C_8$acyl and $R_2$ is e.g. $C_1$-$C_{12}$alkylene.

These compounds are effective stabilisers for organic materials, in particular synthetic polymers, against the actions of light, heat and oxidation.

EP 0 518 807 A1

Jouve, 18, rue Saint-Denis, 75001 PARIS

The present invention relates to novel piperidine compounds, to their use as light stabilisers, heat stabilisers and oxidation stabilisers for organic materials, in particular synthetic polymers, and to organic materials thus stabilised.

Numerous patent on the preparation of 2,2,6,6-tetramethyl-4-piperidinol derivatives and on the use thereof as light stabilisers for synthetic polymers have been published, in particular US Patent 3 640 928, 3 984 371, 4 021432, 4 618 634 and 4 668 722.

The present invention relates to novel piperidine compounds of the formula (I)

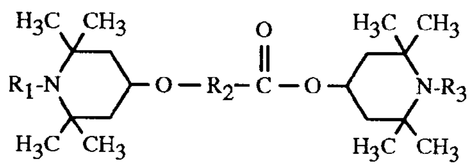

(I)

in which $R_1$ and $R_3$ which can be identical or different are hydrogen, $C_1$-$C_8$alkyl, O, OH, NO, $CH_2CN$, $C_1$-$C_{18}$alkoxy, $C_5$-$C_{12}$cycloalkoxy, $C_3$-$C_6$alkenyl, $C_7$-$C_9$phenylalkyl which is unsubstituted or mono-, di- or tri-substituted on the phenyl by $C_1$-$C_4$alkyl; or $C_1$-$C_8$acyl, $R_2$ is $C_1$-$C_{12}$alkylene, $C_2$-$C_{18}$alkylidene or one of the groups of the formulae (IIa)-(IIe)

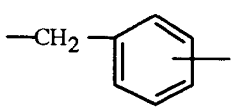 ,

(IIa)

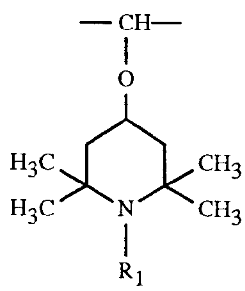 ,

(IIb)

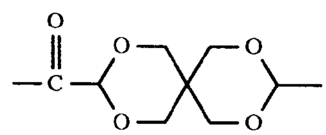 ,

(IIc)

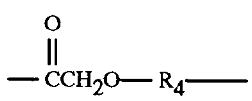 ,

(IId)

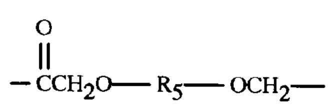

(IIe)

in which $R_1$ is as defined above, $R_4$ is methylene or phenylene and $R_5$ is $C_2$-$C_{12}$alkylene, $C_4$-$C_{12}$alkylene interrupted by 1, 2 or 3 oxygen atoms; $C_5$-$C_7$cycloalkylene, $C_5$-$C_7$cycloalkylenedi($C_1$-$C_4$alkylene), $C_2$-$C_4$alkylidenedi($C_5$-$C_7$cycloalkylene), phenylene, phenylenedi($C_1$-$C_4$alkylene), $C_1$-$C_4$alkylenediphenylene or $C_2$-$C_4$Alkylidenediphenylene, where each phenylene group is unsubstituted or mono- or di-substituted by $C_1$-$C_4$alkyl; or $R_5$ is a group of the formula (IIIa) or (IIIb)

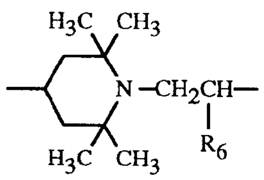

,

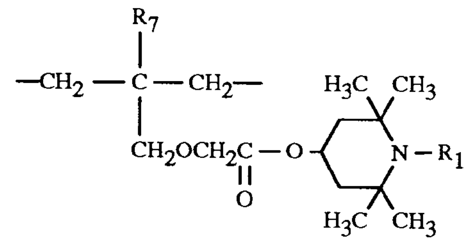

(IIIa)

(IIIb)

in which $R_6$ is hydrogen or $C_1$-$C_4$alkyl and $R_7$ is hydrogen, $C_1$-$C_4$alkyl or a group of the formula (IV)

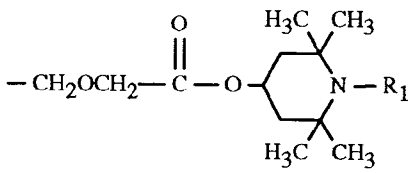

(IV).

Examples of alkyl having not more than 8 carbon atoms are methyl, ethyl, propyl, isopropyl, butyl, isobutyl, t-butyl, pentyl, hexyl, heptyl and octyl. Methyl is particularly preferred.

Examples of $C_1$-$C_{18}$alkoxy are methoxy, ethoxy, propoxy, isopropoxy, butoxy, isobutoxy, pentoxy, isopentoxy, hexoxy, heptoxy, octoxy, decyloxy, dodecyloxy, tetradecyloxy, hexadecyloxy and octadecyloxy. Preferred examples are $C_6$-$C_{12}$alkoxy, in particular heptoxy and octoxy.

Representative examples of $C_5$-$C_{12}$cycloalkoxy $R_1$ and $R_3$ are cyclopentoxy, cyclohexoxy, cycloheptoxy, cyclooctoxy, cyclodecyloxy and cyclododecyloxy. Cyclopentoxy and cyclohexoxy are preferred.

Examples of $C_3$-$C_6$alkenyl are allyl, 2-methylallyl, butenyl and hexenyl. Allyl is particularly preferred. The carbon atom being linked to the piperidinyl nitrogen is preferably saturated.

Examples of $C_7$-$C_9$phenylalkyl which is unsubstituted or substituted on the phenyl by $C_1$-$C_4$alkyl are benzyl, methylbenzyl, dimethylbenzyl, trimethylbenzyl, t-butylbenzyl and 2-phenylethyl. Benzyl is preferred.

Acyl $R_1$ and $R_3$ having not more than 8 carbon atoms can be an aliphatic or aromatic group. Representative examples are formyl, acetyl, propionyl, butyryl, pentanoyl, hexanoyl, heptanoyl, octanoyl, benzoyl, acryloyl or crotonyl. $C_1$-$C_8$alkanoyl, $C_3$-$C_8$alkenoyl and benzoyl are preferred. Acetyl is especially preferred.

Examples of alkylene having not more than 12 carbon atoms are methylene, ethylene, propylene, trimethylene, tetramethylene, pentamethylene, 2,2-dimethyltrimethylene, hexamethylene, heptamethylene, octamethylene, decamethylene and dodecamethylene.

Examples of $C_2$-$C_{18}$alkylidene are ethylidene, propylidene, isopropylidene, butylidene, pentylidene, hexylidene, heptylidene, nonylidene, undecylidene, tridecylidene, pentadecylidene and heptadecylidene.

Examples of $C_4$-$C_{10}$alkylene interrupted by 1, 2 or 3 oxygen atoms are 3-oxapentane-1,5-diyl, 3,6-dioxaoctane-1,8-diyl and 3,6,9-trioxaundecane-1,11-diyl.

Representative examples of radicals with 1 or 2 $C_5$-$C_7$cycloalkylene groups are cyclohexylene, cyclohexylenedimethylene and isopropylidenedicyclohexylene.

Representative examples of radicals with 1 or 2 unsubstituted or substituted phenylene groups are phenylene, methylphenylene, dimethylphenylene, t-butylphenylene, di-t-butylphenylene, xylylene, methylenediphenylene and isopropylidenediphenylene.

The preferred definitions of $R_1$ and $R_3$ are hydrogen, $C_1$-$C_4$alkyl, OH, $C_6$-$C_{12}$alkoxy, $C_5$-$C_8$cycloalkoxy, allyl, benzyl and acetyl, in particular hydrogen, methyl, octoxy, cyclohexoxy, allyl and benzyl.

Those compounds of the formula (I) are preferred in which $R_2$ is $C_1$-$C_{10}$alkylene, $C_2$-$C_{17}$alkylidene or one of the groups of the formulae (IIa)-(IIe) in which $R_4$ is methylene or phenylene and $R_5$ is $C_2$-$C_{10}$alkylene, $C_4$-$C_{10}$alkylene interrupted by 1, 2 or 3 oxygen atoms; cyclohexylene, cyclohexylenedimethylene, isopropylidenedicyclohexylene, phenylene, xylylene, methylenediphenylene, isopropylidenediphenylene or a group of the formula (IIIa) or (IIIb) in which $R_6$ is hydrogen or methyl and $R_7$ is hydrogen, methyl, ethyl or a group of the formula (IV).

Those compounds of the formula (I) are particularly preferred in which $R_2$ is $C_1$-$C_8$alkylene, $C_2$-$C_{15}$alkyli-

dene or one of the groups of the formulae (IIa)-(IIe) in which $R_4$ is methylene or phenylene and $R_5$ is $C_2$-$C_8$alkylene, $C_4$-$C_8$alkylene interrupted by 1, 2 or 3 oxygen atoms; cyclohexylenedimethylene, isopropylidenedicyclohexylene, phenylene, xylylene, isopropylidenediphenylene or a group of the formula (IIIa) or (IIIb) in which $R_6$ is hydrogen or methyl and $R_7$ is methyl, ethyl or a group of the formula (IV).

Those compounds of the formula (I) are of special interest in which $R_2$ is $C_1$-$C_5$alkylene, $C_2$-$C_{11}$ alkylidene or one of the groups of the formulae (IIa)-(IIe) in which $R_4$ is methylene and $R_5$ is $C_2$-$C_6$alkylene, $C_4$-$C_6$alkylene interrupted by 1 or 2 oxygen atoms; or a group of the formula (IIIa) or (IIIb) in which $R_6$ is hydrogen or methyl and $R_7$ is methyl or a group of the formula (IV).

Those compounds of the formula (I) are of particular interest in which $R_1$ and $R_3$ which can be identical or different are hydrogen, methyl, octoxy, cyclohexoxy, allyl or benzyl and $R_2$ is methylene or one of the groups of the formulae (IIa)-(IId) in which $R_4$ is methylene.

Examples of particularly preferred compounds of the formula (I) are those of the formulae

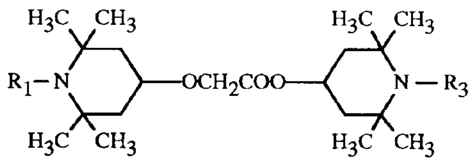
,
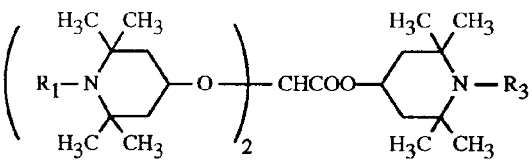
,

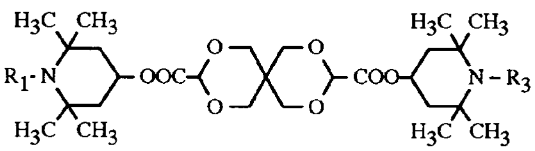
,
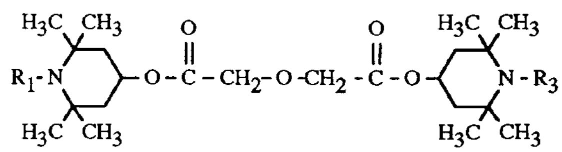

The compounds of the present invention can be prepared by diverse processes known per se.

If $R_2$ is $C_1$-$C_{12}$alkylene, $C_2$-$C_{18}$alkylidene or a group of the formula (IIa), the compounds of the formula (I) can be prepared, for example, by reacting a compound of the formula (V)

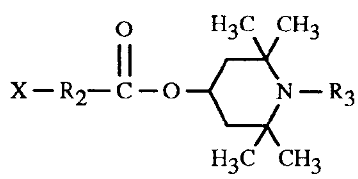
(V)

where X is e.g. Cl or Br, with a compound of the formula (VI)

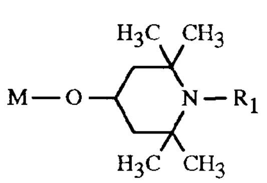
(VI)

where M is Li, Na or K.

If $R_2$ is a group of the formula (IIb), the compound of the formula (I) can be prepared, for example, by reacting a compound of the formula (VII)

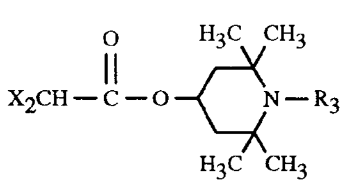
(VII)

with 2 mol of a compound of the formula (VI) or, if $R_1 = R_3$, e.g. by reacting glyoxylic acid or a $C_1$-$C_4$alkyl glyoxylate or a $C_1$-$C_4$alkyl di($C_1$-$C_4$alkoxy)acetate with 3 mol of a compound of the formula (VIII)

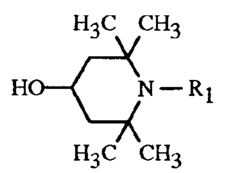

(VIII)

in the presence of an organic or inorganic acidic catalyst, for example benzenesulfonic acid, p-toluenesulfonic acid, methanesulfonic acid, HCl or $H_2SO_4$.

If $R_2$ is a group of the formula (IIc), (IId) or (IIe), the compounds of the formula (I) can be prepared, for example, by reacting 2 mol of a compound of the formula (VIII) with 1 mol of a compound of the formula (IXa), (IXb) or (IXc)

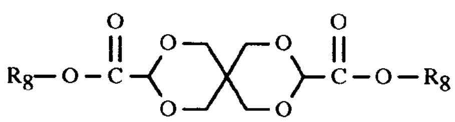 , 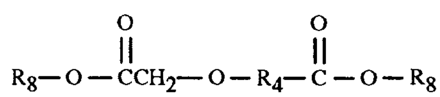 ,

(IXa) (IXb)

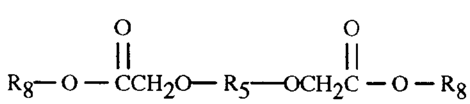

(IXc)

in which $R_8$ is $C_1$-$C_4$alkyl, in the presence of a transesterification catalyst, for example a Ti alkoxide or a hydride, alkoxide or amide of Li, Na or K.

The various reactions can be carried out, for example, in a hydrocarbon solvent, e.g. toluene or xylene, or, in the case of reacting a halogen ester of the formula (V) or (VII) with a compound of the formula (VI) in which M is K or Na, can be carried out in a mixture of toluene or xylene with a tertiary $C_4$-$C_6$alkanol, preferably 2-methyl-2-butanol, operating at a temperature between e.g. 0° and 200°C, preferably between 20° and 150°C.

The compounds of the formula (V) and (VII) are conveniently prepared by transesterification of, respectively, a halogen ester X-$R_2$-$COOR_8$ or $X_2$-CH-$COOR_8$ with an alcohol of the formula (VIII) in appropriate molar ratios and in the presence of a transesterification catalyst selected from the group defined above; the resulting compounds can be utilised directly for the subsequent reactions.

The compounds of the formula (VI) are prepared, for example, by reaction of an alcohol of the formula (VIII) with Li, Na or K in toluene or xylene, with heating under reflux.

If M is K or Na, the compounds of the formula (VI) are preferably prepared by reaction of an alcohol of the formula (VIII) with NaOH or KOH, operating in a tertiary $C_4$-$C_6$alkanol, preferably 2-methyl-2-butanol, or in a mixture of the said alcohol with a hydrocarbon solvent such as hexane, cyclohexane, benzene, toluene or xylene, preferably hexane or toluene, by heating under reflux while azeotropically separating off the water of reaction. This method of preparing the compounds of the formula (VI) is novel and constitutes a subject of the present invention.

The compounds of the formula (VIII) are prepared, for example, by catalytic reduction of the corresponding 4-piperidones or by introducing, using the appropriate reagent, the group $R_1$ (for $R_1 \neq H$) into the 1-position of 2,2,6,6-tetramethyl-4-piperidinol. The compounds of the formula (IXa) are prepared e.g. according to Clement, J. Org. Chem. 24, 1958 (1959).

The compounds of the formulae (IXb) and (IXc) are easily obtainable by known processes, starting from commercially available product.

As mentioned at the outset, the novel compounds of the present invention are highly effective in improving the light stability, heat stability and oxidation stability of organic materials, in particular synthetic polymers and

copolymers.

Examples of such organic materials which can be stabilised are:

1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE), linear low density polyethylene (LLDPE), branched low density polyethylene (BLDPE).

2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).

3. Copolymers of monoolefines and diolefines with each other or with other vinyl monomers, such as, for example, ethylene/propylene, linear low density polyethylene (LLDPE) and it mixtures with low density polyethylene (LDPE), propylene/butene-1,ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate copolymers and their copolymers with carbon monoxide or ethylene/acrylic acid copolymers and their salt (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/ethylene-vinyl acetate copolymers (EVA), LDPE/ethylene-acrylic acid copolymers (EAA), LLDPE/EVA, LLDPE/EAA and statistical or alternating polyalkylene/carbon monoxide-copolymers as well as their mixtures with other polymers, for example polyamide.

3a. Hydrocarbon resins (for example $C_5$-$C_9$) and hydrogenated modifications thereof (for example tackyfiers) and mixtures of polyalkylenes and starch.

4. Polystyrene, poly-(p-methylstyrene), poly-(α-methylstyrene).

5. Copolymers of styrene or α-methylstyrene with dienes or acrylic derivatives, such as, for example, styrene/butadiene, styrene/acrylonitrile, styrene/alkyl methacrylate, styrene/maleic anhydride, styrene/butadiene/alkyl acrylate, styrene/butadiene/alkyl methacrylate styrene/acrylonitrile/methyl acrylate; mixtures of high impact strength from styrene copolymers and another polymer, such as, for example, from a polyacrylate, a diene polymer or an ethylene/propylene/diene terpolymer, and block copolymers of styrene, such as, for example, styrene/butadiene/styrene, styrene/isoprene/styrene, styrene/ethylene/butylene/styrene or styrene/ethylene/propylene/ styrene.

6. Graft copolymers of styrene or α-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.

7. Halogen-containing polymers, such as polychloroprene, chlorinated rubbers, chlorinated or sulfochlorinated polyethylene, copolymers of ethylene and chlorinated ethylene, epichlorohydrin homo- and copolymers, polymers from halogen-containing vinyl compounds,as for example, poly- vinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.

8. Polymers which are derived from α,β-unsaturated acids and derivatives thereof, such as polyacrylates and polymethacrylates, with butyl acrylate impact resistant modified polymethyl methacrylate, polyacrylamide and polyacrylonitrile.

9. Copolymers from the monomers mentioned under 8) with each other or with other unsaturated monomers, such as, for instance, acrylonitrile/ butadiene, acrylonitrile/alkyl acrylate, acrylonitrile/alkoxyalkyl acrylate or acrylonitrile/vinyl halogenide copolymers or acrylonitrile/ alkyl methacrylate/butadiene terpolymers.

10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.

11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as a comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corre- sponding lactams, such as polyamide 4, polyamide 6, polyamide 6/6, 6/10, 6/9, 6/12, 4/6, 12/12 polyamide 11, polyamide 12, aromatic polyamides obtained by condensation of m-xylene diamine and adipic acid; polyamides prepared from hexamethylenediamine and isophthalic or/and terephthalic acid and optionally an elastomer as modifier, for example poly-2,4,4,-trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols andl[ch]or from hydroxycarboxylic acids or the corresponding lactones, such as polyethylene terephthalate, polybutylene terephthalate, poly-1,4-dimethylolcyclohexane terephthalate, poly-[2,2,-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agent, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agent.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose; rosins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fat and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.
29. Aqueous emulsions of natural or synthetic rubber, e.g. natural latex or latices of carboxylated styrene/butadiene copolymers.

The compounds of the formula (I) are particularly suitable for improving the light stability, heat stability and oxidation stability of polyolefins, especially polyethylene and polypropylene.

The compounds of the formula (I) can be used in mixtures with organic materials in various proportions depending on the nature of the material to be stabilised, on the end use and on the presence of other additives.

In general, it is appropriate to use, for example, 0.01 to 5 % by weight of the compounds of the formula (I), relative to the weight of the material to be stabilised, preferably between 0.05 and 1 %.

In general, the compounds of the formula (I) can be added to the polymeric materials before, during or after the polymerization or crosslinking of the said materials.

The compounds of the formula (I) can be incorporated in the polymeric materials in the pure form or encapsulated in waxes, oils or polymers.

The compounds of the formula (I) can be incorporated in the polymeric materials by various processes, such as dry mixing in the form of powder, or wet mixing in the form of solutions or suspensions or also in the form of a masterbatch; in such operations, the polymer can be used in the form of powder, granules, solutions, suspensions or in the form of latices.

The materials stabilised with the products of the formula (I) can be used for the production of mouldings, films, tapes, monofilament, fibres, surface coatings and the like.

If desired, other conventional additives for synthetic polymers, such as antioxidant, UV absorbers, nickel stabilisers, pigments, fillers, plasticisers, antistatic agent, flameproofing agents, lubricant, corrosion inhibitors and metal deactivators, can be added to the mixtures of the compounds of the formula (I) with the organic materials.

Particular examples of additives which can be used in admixture with the compounds of the formula (I) are:

1. Antioxidant

1.1. Alkylated monophenols, for example 2,6-di-tert-butyl-4-methyl-phenol, 2-tert-butyl-4,6-dimethylphenol, 2,6-di-tert-butyl-4-ethylphenol, 2,6-di-tert-butyl-4-n-butylphenol, 2,6-di-tert-butyl-4-iso-butylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexyl-phenol, 2,6-di-tert-butyl-4-methoxymethylphenol, 2,6-di-nonyl-4-methylphenol, 2, 4-dimethyl-6-(1′-methyl-undec-1′-yl)-phenol, 2,4-dimethyl-6-(1′-methylheptadec-1′-yl)-phenol, 2,4-dimethyl-6-(1′-methyl-tridec-1′-yl)-phenol and mixtures therof.

1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.

1.3. Hydroquinones and alkylated hydroquinones, for example 2,6-di-tert-butyl-4-methoxy-phenol, 2,5-di-tert-butylhydroquinone, 2,5-di-tert-amylhydroquinone, 2,6-diphenyl-4-octadecyloxyphenol, 2,6-di-tert-butyl-hydroquinone, 2,5-di-tert-butyl-4-hydroxyanisole, 3,5-di-tert-butyl-4-hydroxyanisole, 3,5-di-tert-butyl-4-hydroxyphenyl-stearate, bis-(3,5-di-tert-butyl-4-hydroxyphenyl)adipate.

1.4. Hydroxylated thiodiphenyl ethers, for example 2,2′-thiobis(6-tert-butyl-4-methylphenol), 2,2′-thiobis(4-octylphenol), 4,4′-thiobis(6-tert-butyl-3-methylphenol), 4,4′-thiobis(6-tert-butyl-2-methylphenol), 4,4′-thio-bis-(3,6-di-sec-amylphenol), 4,4′-bis-(2,6-dimethyl-4-hydroxyphenyl)-disulfide.

1.5. Alkylidenebisphenols, for example 2,2′-methylenebis(6-tert-butyl-4-methylphenol), 2,2′-methylene-bis(6-tert-butyl-4-ethylphenol), 2,2′-methylenebis(4-methyl-6-(α-methylcyclohexyl)phenol], 2,2′-methylene-bis(4-methyl-6-cyclohexylphenol), 2,2′-methylenebis(6-nonyl-4-methylphenol), 2,2′-methylenebis(4,6-di-tert-butylphenol), 2,2′-ethylidenebis(4,6-di-tert-butylphenol), 2,2′-ethylidenebis(6-tert-butyl-4-isobutylphenol), 2, 2′-methylenebis[6-(α-methylbenzyl)-4-nonylphenol], 2,2′-methylenebis[6-(α,α-dimethylbenzyl)-4-nonylphenol], 4,4′-methylenebis(2,6-di-tert-butylphenol), 4,4′-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1, 3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis(3′-tert-butyl-4′-hydroxyphenyl)butyrate], bis(3-tert-butyl-4-hydroxy-5-methylphenyl)dicyclopentadiene, bis[2-(3′-tert-butyl-2′-hydroxy-5′-methylbenzyl)-6-tertbutyl- 4-methylphenyl]terephthalate, 1,1-bis-(3,5-dimethyl-2-hydroxyphenyl)butan, 2,2-bis-(3,5-di-tert.-butyl-4-hydroxyphenyl)-propan, 2,2-bis-(5-tert.-butyl-4-hydroxy-2-methylphenyl)-4-n-dodecylmercapto-butan, 1,1,5,5-tetra-(5-tert.-butyl-4-hydroxy-2-methylphenyl)-pentan.

1.6.O-, N- and S-benzyl compounds, for example 3,5,3′,5′-tetra-tert.-butyl-4,4′-dihydroxydibenzylether, octadecyl-4-hydroxy-3,5-dimethylbenzyl-mercaptoacetate, tris-(3,5-di-tert.-butyl-4-hydroxybenzyl)-amine, bis-(4-tert.-butyl-3-hydroxy-2,6-dimethylbenzyl)dithioterephthalate, bis-(3,5-di-tert.-butyl-4-hydroxybenzyl)-sulfide, isooctyl-3,5-di-tert.-butyl-4-hydroxybenzyl-mercaptoacetate.

1.7. Hydroxybenzylated Malonates, for example
dioctadecyl-2,2-bis-(3,5-di-tert.-butyl-2-hydroxybenzyl)-malonate, di-octadecyl-2(3-tert.-butyl-4-hydroxy-5-methylbenzyl)-malonate, di-dodecylmercaptoethyl-2,2-bis-(3,5-di-tert.-butyl-4-hydroxybenzyl)-malonate, Di-[4-(1,1,3,3-tetramethylbutyl)-phenyl]2,2-bis-(3,5-di-tert.-butyl-4-hydroxybenzyl)-malonate.

1.8. Hydroxybenzyl-Aromatics, for example 1,3,5-tris-(3,5-di-tert.-butyl-4-hydroxybenzyl)-2,4,6-trimethylbenzene, 1,4-bis-(3,5-di-tert.-butyl-4-hydroxybenzyl)-2,3,5,6-tetramethylbenzene, 2,4,6-tris-(3,5-di-tert.-butyl-4-hydroxybenzyl)-phenol.

1.9. Triazine Compounds, for example

2,4-bis-octylmercapto-6-(3,5-di-tert.-butyl-4-hydroxyanilino)-1,3,5-triazine, 2-octylmercapto-4,6-bis-(3,5-di-tert.-butyl-4-hydroxyanilino)-1,3,5-triazine, 2-octylmercapto-4,6-bis-(3,5-di-tert.-butyl-4-hydroxyphenoxy)-1,3,5-triazine, 2,4,6-tris-(3,5-di-tert.-butyl-4-hydroxyphenoxy)-1,2,3-triazine, 1,3,5-tris-(3,5-di-tert.-butyl-4-hydroxybenzyl)-isocyanurate, 1,3,5-tris-(4-tert.-butyl-3-hydroxy-2,6-dimethylbenzyl)-isocyanurate, 2,4,6-tris-(3,5-di-tert.-butyl-4-hydroxyphenylethyl)-1,3,5-triazine, 1,3,5-tris-(3,5-di-tert.butyl-4-hydroxyphenylpropionyl)-hexahydro-1,3,5-triazine, 1,3,5-tris-(3,5-dicyclohexyl-4-hydroxybenzyl)-isocyanurate.

1.10. Benzylphosphonates, for example dimethyl-2,5-di-tert.-butyl-4-hydroxybenzylphosphonate, diethyl-3,5-di-tert.-butyl-4-hydroxybenzylphosphonate, dioctadecyl-3,5-di-tert.-butyl-4-hydroxybenzylphosphonate, dioctadecyl-5-tert.-butyl-4-hydroxy-3-methylbenzylphosphonate, Ca-salt of the 3,5-di-tert.-butyl-4-hydroxybenzyl-phosphonic acid monoethylester.

1.11. Acylaminophenols, for example lauric acid 4-hydroxyanilide, stearic acid 4-hydroxyanilide, octyl N-(3,5-di-tert-butyl-4-hydroxyphenyl)-carbamate.

1.12. Esters of β-(3,5-di-tert-butyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo[2[.2.2]-octane.

1.13. Esters of β-(5-tert-butyl-4-hydroxy-3-methylphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo[2.2.2]-octane.

1.14 Esters of β-(3,5-dicyclohexyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo[2.2.2]-octane.

1.15 Esters of 3,5-di-tert.-butyl-4-hydroxyphenyl acetic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo[2.2.2]-octane.

1.16. Amides of β-(3,5-di-tert-butyl-4-hydroxyphenyl)propionic acid e.g. N,N′-bis(3,5-di-tert-butyl-4-hydroxyphenylpropionyl)hexamethylene-diamine, N,N′-bis(3,5-di-tert-butyl-4-hydroxyphenylpropionyl)tri-methylene-diamine, N,N′-bis(3,5-di-tertbutyl-4-hydroxyphenylpropionyl)-hydrazine.

2. UV absorbers and light stabilisers

2.1.2-(2′-Hydroxyphenyl)benzotriazoles, for example the 5′-methyl, 3′,5′-di-tert-butyl, 5′-tert-butyl, 5′-(1,1,3,3-tetramethylbutyl), 5-chloro-3′,5′-di-tert-butyl, 5-chloro-3′-tert-butyl-5′-methyl, 3′-sec-butyl-5′-tert-butyl, 4′-octoxy, 3′,5′-di-tert-amyl and 3′,5′-bis(α,α-dimethylbenzyl), mixture of 5-chloro-3′-tert.-butyl-5′-(2-octyloxycarbonylethyl)-and 5-chloro-3′-tert.-butyl-5′-[2-(2-ethylhexyloxy)-carbonylethyl]-, 5-chloro-3′-tert.-butyl-5′-(2-methoxycarbonylethyl)-, 3′-tert.-butyl-5′-(2-methoxycarbonylethyl)-, 3′-tert.-butyl-5′-(2-octyloxycarbonylethyl)-, 3′-tert.-butyl-5′-[2-(2-ethylhexyloxy)carbonylethyl]-, 3′-dodecyl-5′-methyl- and 3′-tert.-butyl-5′-(2-isooctyloxycarbonylethyl)-2′-hydroxyphenyl-2H-benztriazole(2), 2,2′-methylene-bis[4-(1,1,3,3-tetramethylbutyl)6-benztriazole-2-yl-phenol]; product of ester interchange of 2-[3′-tert.-butyl-5′-(2-methoxycarbonylethyl)-2′-hydroxy-phenyl]-2H-benztriazole with polyethylene glycol 300; $[R-CH_2CH_2-COO(CH_2)_3]_2$ with rR=3′-tert.-butyl-4′-hydroxy-5′-2H-benzotriazole-2-yl-phenyl.

2.2.2-Hydroxybenzophenones, for example the 4-hydroxy, 4-methoxy, 4-octoxy, 4-decyloxy, 4-dodecyloxy, 4-benzyloxy, 4,2′,4′-trihydroxy and 2′-hydroxy-4,4′-dimethoxy derivatives.

2.3. Esters of substituted and unsubstituted benzoic acids, as for example 4-tert.butylphenyl salicylate, phenyl salicylate, octylphenyl salicylate, dibenzoylresorcinol, bis-(4-tert.butylbenzoyl)-resorcinol, benzoylresorcinol, 2,4-di-tert.butylphenyl 3,5-di-tert.butyl-4-hydroxybenzoate, hexadecyl 3,5-di-tert.butyl-4-hydroxybenzoate, octadecyl 3,5-di-tert.-butyl-4-hydroxybenzoate, 2 methyl-4,6-di-tert.-butylphenyl 3,5-di-tert.-butyl-4-hydroxybenzoate.

2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxy-cinnamate, methyl α-carbomethoxy-p-methoxycinnamate and N-(β-carbomethoxy-β-cyanovinyl)-2-methylindoline.

2.5. Nickel compounds, for example nickel complexes of 2,2′-thio-bis-[4-( 1, 1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salt of 4-hydroxy-3,5-di-tert-butylbenzylphosphonic acid monoalkyl esters, e.g. of the methyl or ethyl ester, nickel complexes of ketoximes, e.g. of 2-hydroxy-4-methyl-phenyl undecyl ketoxime, nickel complexes of 1-phenyl-4-lauroyl-5-hydroxypyrazole, with or without additional ligands.

2.6. Sterically hindered amines, for example bis(2,2,6,6-tetramethyl-piperidyl) sebacate, bis-(2,2,6,6-tetramethyl-piperidyl) succinate, bis(1,2,2,6,6-pentamethylpiperidyl) sebacate, bis(1,2,2,6,6-pentamethylpiperidyl) n-butyl-3,5-di-tert-butyl-4-hydroxy-benzylmalonate, the condensation product of 1-(2-hydroxyethyl)-2,2,6,6-tetramethyl-4-hydroxypiperidine and succinic acid, the condensation product of N,N′-bis(2,2,6,6-tetramethyl-4-piperidyl)hexamethylenediamine and 4-tert-octylamino-2,6-dichloro- 1,3,5-triazine, tris(2,2,6,6-tetramethyl-4-piperidyl) nitrilotriacetate, tetrakis(2,2,6,6-tetramethyl-4-piperidyl)-1,2,3,4-butane-tetracarboxylate, 1,1′-(1,2-ethanediyl)bis-(3,3,5,5-tetramethylpiperazinone), 4-benzoyl-2,2,6,6-tetramethylpiperidine, 4-stearyloxy-2,2,6,6-tetramethylpiperidine, bis-( 1,2,2,6,6-pentamethylpiperidyl)-2-n-butyl-2-(2-hydroxy-3,5-di-tert.-butyl-benzyl) malonate, 3-n-octyl-7,7,9,9-tetramethyl-1,3,8-triazasprio[4.5]decan-2,4-dion, bis-(1-octyloxy-2,2,6,6-tetramethylpiperidyl) sebacate, bis-(1-octyloxy-2,2,6,6-tetramethylpiperidyl) succinate, product of condensation of N,N′-bis-(2,2,6,6-tetramethyl-4-piperidyl)-hexamethylene diamine and 4-morpholino-2,6-dichloro-1,3,5-triazine, product of condensation of-chloro-4,6-di-(4-n-butylamino-2,2,6,6-tetramethylpiperidyl)-1,3,5-triazine and 1,2-bis-(3-aminopropylamino)ethane, product of condensation of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dion, 3-dodecyl-1-(2,2,6,6-tetramethyl-4-piperidyl)pyrrolidin-2,5-dion, 3-dodecyl-1-( 1,2,2,6,6-pentamethyl-4-piperidyl)-pyrrolidin-2,5-dion.

2.7. Oxalic acid diamides, for example 4,4′-dioctyloxyoxanilide, 2,2′-dioctyloxy-5,5′-di-tert-butyloxanilide, 2,2′-didodecyloxy-5,5′-di-tert-butyloxanilide, 2-ethoxy-2′-ethyloxanilide, N,N′-bis(3-dimethylamino-propyl)oxalamide, 2-ethoxy-5-tertbutyl-2′-ethyloxanilide and its mixture with 2-ethoxy-2′-ethyl-5,4′-di-tert-butyloxanilide and mixtures of ortho- and para-methoxy-disubstituted oxanilides and mixtures of o- and p-ethoxy-disubstituted oxanilides.

2.8.2-(2-Hydroxyphenyl)-1,3,5-triazines, for example 2,4,6-tris(2-hydroxy-4-octyloxyphenyl)-1,3,5-triazine, 2-(2-hydroxy-4-octyloxy-phenyl)-4,6-bis(2,4-dimethylphenyl)1,3,5-triazine, 2-(2,4-dihydroxy-phenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxy-propyloxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine.

3. Metal deactivators, for example N,N′-diphenyloxalic acid diamide, N-salicylal-N′-salicyloylhydrazine, N,N′-bis(salicyloyl)hydrazine, N,N′-bis(3,5-di-tert-butyl-4-hydroxyphenylpropionyl)hydrazine , 3-salicyl-oylamino-1,2,4-triazole, bis(benzylidene)oxalodihydrazide, Oxanilide, isophthalic acid dihydrazide, sebacic acid-bis-phenylhydrazide, N,N′-diacetal-adipinic acid dihydrazide, N,N′-bis-salicyloyl-oxalic acid dihydrazide, N,N′-bis-salicyloyl-thiopropionic acid dihydrazide.

4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diiso- decyl pentaerythritol diphosphite, bis(2,4-di-tert-butylphenyl) pentaerythritol diphosphite, bis-(2,6-di-tert.-butyl-4-methylphenyl)-pentaeryt hritol diphosphite, bis-isodecyloxy-pentaerythritol diphosphite, bis-(2,4-di-tert.-butyl-6-methylphenyl)-pentaerythritol diphosphite, bis-(2,4,6-tri-tert.-butylphenyl)-pentaerythritol diphsophite, tristearyl sorbitol triphosphite, tetrakis(2,4-di-tert-butylphenyl) 4,4′-biphenylene diphosphonite, 6-isooctyloxy-2,4,8,10-tetra-tert.-butyl-12H-dibenz[d,g]-1,3,2-dioxaphosphocin, 6-fluoro-2,4,8,10-tetra-tert.-butyl-12-methyl-dibenz[d,g]-1,3,2-dioxaphosphocin.

4a. Hydroxylamines, for example dibenzylhydroxylamine, dioctylhydroxylamine, didodecylhydroxylamine, ditetradecylhydroxylamine, dihexadecylhydroxylamine, dioctadecylhydroxylamine, 1-hydroxy-2,2,6,6-tetramethyl-4-piperidyl benzoate or bis(1-hydroxy-2,2,6,6-tetramethyl-4-piperidyl) sebacate.

5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecyl-mercapto)propionate.

6. Polyamide stabilisers, for example, copper salt in combination with iodides and/or phosphorus compounds and salts of divalent manganese.

7. Basic co-stabilisers, for example, melamine, polyvinylpyrrolidone, dicyandiamide, triallyl cyanurate, urea

derivatives, hydrazine derivatives, amines, polyamides, polyurethanes, alkali metal salt and alkaline earth metal salt of higher fatty acids for example Ca stearate, Zn stearate, Mg behenate, Mg stearate, Na ricinoleate and K palmitate, antimony pyrocatecholate or zinc pyrocatecholate.

8. Nucleating agent, for example, 4-tert.butyl-benzoic acid, adipic acid, diphenylacetic acid.

9. Fillers and reinforcing agent, for example, calcium carbonate, silicates, glass fibres, asbestos, talc, kaolin, mica, barium sulfate, metal oxides and hydroxydes, carbon black, graphite.

10. Other additives, for example, plasticisers, lubricants, emulsifiers, pigment, optical brighteners, flameproofing agent, antistatic agents and blowing agents.

The compounds of the present invention can also be used as stabilisers, especially as light stabilisers, for almost all materials known in the art of photographic reproduction and other reproduction techniques as e.g. described in Research Disclosure 1990, 31429 (page 474 to 480).

Several examples of the preparation of the compounds of the formula (I) are reported for more detailed illustration of the present invention; these examples are given solely for illustrative purposes and do not imply any restriction. Examples 1, 7, 9 and 10 disclose especially preferred compounds of the instant invention.

Example 1:

Preparation of the compound of the formula

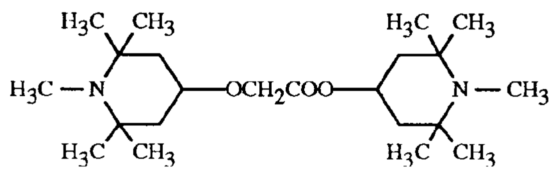

A solution comprising 80.5 g (0.47 mol) of 1,2,2,6,6-pentamethyl-4-piperidinol, 78.5 g (0.47 mol) of ethyl bromoacetate and 0.6 ml of Ti(IV) isopropoxide in 300 ml of toluene is heated for 4 hours at the reflux, slowly distilling off the solvent together with the ethanol set free during the reaction and substituting it with an equal volume of fresh toluene.

After cooling to ambient temperature, the reaction mixture is washed with water and dried over $Na_2SO_4$.

A solution comprising 90.8 g (0.47 mol) of the sodium salt of 1,2,2,6,6-pentamethyl-4-piperidinol in 500 ml of toluene (obtained by reacting 10.8 g of sodium with 80.5 g of 1,2,2,6,6-pentamethyl-4-piperidinol in 500 ml of toluene) is added slowly to the toluene solution, thus obtained, of 1,2,2,6,6-pentamethyl-4-piperidyl bromoacetate, without exceeding 30°C.

The mixture is stirred for 2 hours at ambient temperature and, after washing with water, drying over $Na_2SO_4$ and evaporation of the solvent, the residue is distilled in vacuo. The resulting product has a boiling point of 180°C/2.7 mbar.

| Analysis for $C_{22}H_{42}N_2O_3$ | |
|---|---|
| Calculated: | C = 69.07 %; H = 11.06 %; N = 7.32 % |
| Found: | C = 68.58 %; H = 10.89 %; N = 7.21 % |

Example 2:

The same compound as in Example 1 is prepared as described therein, but with replacement of the toluene solution comprising the sodium salt of 1,2,2,6,6-pentamethyl-4-piperidinol by a solution of 98.4 g (0.47 mol) of the potassium salt of 1,2,2,6,6-pentamethyl-4-piperidinol, obtained by heating 26.4 g of potassium hydroxide and 80.5 g of 1,2,2,6,6-pentamethyl-4-piperidinol under reflux in 400 ml of 2-methyl-2-butanol and 70 ml of toluene, while separating off azeotropically the water of reaction.

Examples 3-6:

Following the procedure described in Example 1, but using the respective reagent in the appropriate molar ratios, the following compounds of the formula

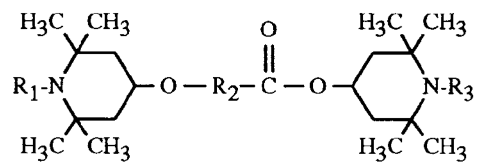

are prepared.

| Example | $R_1$ | $R_2$ | $R_3$ | m.p. (°C) |
|---|---|---|---|---|
| 3 | $CH_2{=}CH{-}CH_2{-}$ | $-CH_2-$ | $CH_2{=}CH{-}CH_2{-}$ | 69-71 |
| 4 | 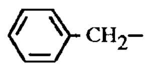 | $-CH_2-$ | 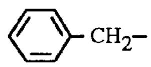 | 141-143 |
| 5 | $-CH_3$ | 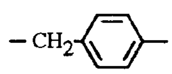 | $-CH_3$ | 72-74 |
| 6 | 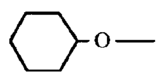 | $-CH_2-$ | 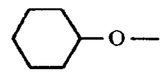 | wax |

Example 7:

Preparation of the compound of the formula

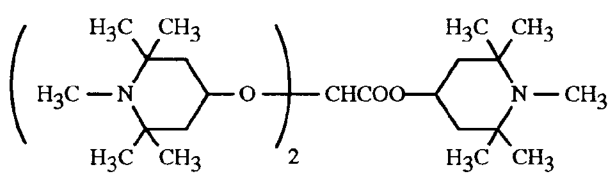

A solution comprising 39.4 g (0.23 mol) of 1,2,2,6,6-pentamethyl-4-piperidinol, 36.1 g (0.23 mol) of ethyl dichloroacetate and 0.5 ml of Ti(IV) isopropoxide in 200 ml of toluene is heated for 4 hours at the reflux, while slowly distilling off the solvent together with the ethanol set free during the reaction and substituting the latter by an equal volume of fresh toluene.

After cooling to ambient temperature, the reaction mixture is washed with water and dried over $Na_2SO_4$.

A solution comprising 88.9 g (0.46 mol) of the sodium salt of 1,2,2,6,6-pentamethyl-4-piperidinol in 500 ml of toluene (obtained by reacting 10.6 g of sodium with 78.8 g of 1,2,2,6,6-pentamethyl-4-piperidinol in 500 ml of toluene) is added slowly to the resulting toluene solution of 1,2,2,6,6-pentamethyl-4-piperidyl dichloroacetate, without exceeding 30°C.

The mixture is stirred for 2 hours at ambient temperature and, after washing with water, drying over $Na_2SO_4$ and evaporation of the solvent, the residue is crystallised from acetonitrile. The product obtained melt at 105-107°C.

| | |
|---|---|
| Analysis for $C_{32}H_{61}N_3O_4$ | |
| Calculated: | C = 69.65 %; H = 11.14 %; N = 7.61 % |
| Found: | C = 69.51 %; H = 10.99 %; N = 7.55 % |

Example 8:

The same compound as in Example 7 is prepared by heating a mixture of 25 g (0.146 mol) of 1,2,2,6,6-pentamethyl-4-piperidinol, 4.4 g (0.048 mol) of glyoxylic acid monohydrate, 30.6 g (0.161 mol) of p-toluenesulfonic acid monohydrate and 100 ml of toluene for 6 hours under reflux, while azeotropically separating off the water.

After cooling to 40°C, a solution of 20.2 g of potassium carbonate in 50 ml of water is added.

The mixture is stirred for 1 hour at ambient temperature, the aqueous phase is removed and the organic phase is washed with water, dried over $Na_2SO_4$ and evaporated. The residue obtained is crystallised from acetonitrile.

Example 9:

Preparation of the compound of the formula

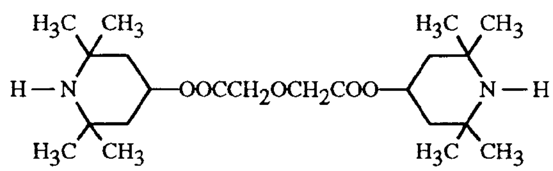

A solution comprising 31.5 g (0.2 mol) of 2,2,6,6-tetramethyl-4-piperidinol, 24.6 g (0.1 mol) of dibutyl diglycolate and 0.7 ml of Ti(IV) isopropoxide in 150 ml of xylene is heated for 5 hours under reflux, while slowly distilling off the solvent together with the butanol set free during the reaction and substituting the latter with an equal volume of fresh xylene. After the end of the reaction, 50 ml of dichloromethane and 100 ml of water are added, and the mixture is stirred for 1 hour at ambient temperature.

The aqueous phase is removed and the organic phase is washed with water, dried over $Na_2SO_4$ and evaporated. The residue is crystallised from n-hexane. The product obtained melt at 118-120°C.

Analysis for $C_{22}H_{40}N_2O_5$

| | |
|---|---|
| Calculated: | C = 64.05 %; H = 9.77 %; N = 6.79 % |
| Found: | C = 64.00 %; H = 9.70 %; N = 6.68 % |

Examples 10-11:

Following the procedure described in Example 9, but using the respective reagent in the appropriate molar ratios, the following compounds of the formula

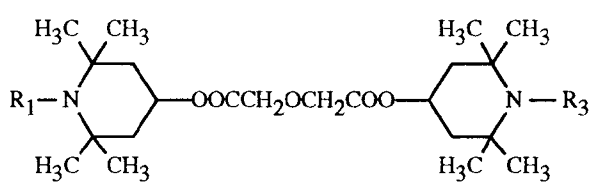

are prepared.

| Example | $R_1/R_3$ | m.p. (°C) |
|---|---|---|
| 10 | $-CH_3$ | 91-93 |
| 11 | 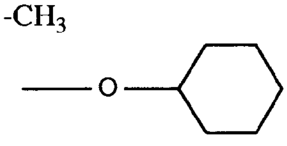 | 63-65 |

Example 12:

Preparation of the compound of the formula

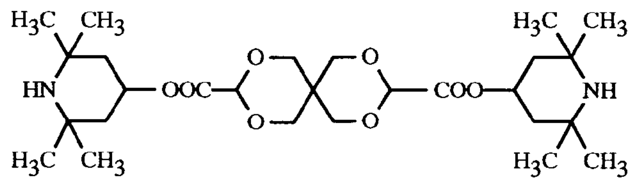

A solution comprising 18.9 g (0.12 mol) of 2,2,6,6-tetramethyl-4-piperidinol, 15.2 g (0.055 mol) of 3,9-bis(methoxycarbonyl)-2,4,8,10-tetraoxaspiro[5.5]undecane and 0.3 ml of Ti(IV) isopropoxide in 100 ml of toluene is heated for 5 hours under reflux, while slowly distilling off the solvent together with the methanol set free during the reaction and substituting the latter by an equal volume of fresh toluene.

After cooling to ambient temperature, the reaction mixture is washed with water, dried over $Na_2SO_4$ and evaporated.

The residue is crystallised from n-hexane. The product obtained melts at 125-126°C

Analysis for $C_{27}H_{46}N_2O_8$

| | |
|---|---|
| Calculated: | C = 61.58 %; H = 8.80 %; N = 5.32 % |
| Found: | C = 61.26 %; H = 8.78 %; N = 5.25 % |

Example 13:

Light-stabilising action in polypropylene tapes.

1 g of each of the compounds indicated in Table 1, 0.5 g of tris(2,4-di-t-butylphenyl) phosphite, 0.5 g of pentaerythritol tetrakis-3-(3,5-di-t-butyl-4-hydroxyphenyl)-propionate and 1 g of calcium stearate are mixed in a slow mixer with 1000 g of polypropylene powder of melt index = 2 g/10 minutes (measured at 230°C and 2.16 kg).

The mixtures are extruded at 200-220°C to give polymer granules which are then converted into stretched tapes of 50 µm thickness and 2.5 mm width, using a pilot-type apparatus (®Leonard-Sumirago (VA) Italy) operating under the following conditions:

| | |
|---|---|
| Extruder temperature: | 210-230°C |
| Head temperature: | 240-260°C |
| Stretch ratio: | 1 : 6 |

The tapes thus prepared are exposed, mounted on a white card, in a 65 WR Weather-O-Meter (ASTM D 2565-85) with a black panel temperature of 63°C. The residual tenacity is measured on samples taken after various times of exposure to light by means of a constant-speed tensometer, the exposure time (in hours) ($T_{50}$) needed to halve the initial tenacity is then calculated.

Tapes prepared under the same conditions as indicated above, but without addition of stabiliser, are exposed for comparison.

The result obtained are shown in Table 1.

Table 1

| Stabiliser | $T_{50}$ (hours) |
|---|---|
| none | 500 |
| Compound from Example 1 | 2300 |
| Compound from Example 3 | 2100 |

Example 14:

Light-stabilising action in polypropylene plaques.

1 g of each of the compounds indicated in Table 2, 0.5 g of tris(2,4-di-t-butylphenyl) phosphite, 0.5 g of pentaerythritol tetrakis-3-(3,5-di-t-butyl-4-hydroxyphenyl)-propionate, 1 g of Phthalocyanine Blue, 1 g of calcium stearate and 1000 g of polypropylene powder of melt index = 2.1 g/10 minutes (measured at 230°C and 2.16 kg) are intimately mixed in a slow mixer.

The mixtures obtained are extruded at a temperature of 200-220°C to give polymer granules which are then converted into plaques of 2 mm thickness by compression-moulding at 190-220°C. The plaques obtained are exposed in a model 65 WR Weather-O-Meter (ASTM G 26-77) with a black panel temperature of 63°C up to the onset of superficial embrittlement (chalking).

A polypropylene plaque prepared under the same conditions as indicated above, but without addition of compounds of the invention, is exposed for comparison.

The exposure time (in hours) needed to reach the onset of superficial embrittlement is shown in Table 2

Table 2

| Stabiliser | Time to embrittlement (hours) |
|---|---|
| none | 550 |
| Compound from Example 7 | 4300 |
| Compound from Example 9 | 4300 |

**Claims**

**1.** A compound of the formula (I)

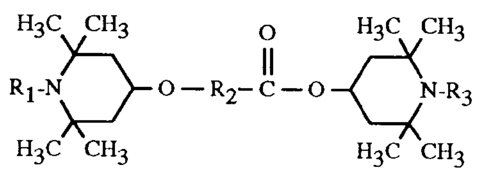

(I)

in which $R_1$ and $R_3$ which are identical or different are hydrogen, $C_1$-$C_8$alkyl, O, OH, NO, $CH_2CN$, $C_1$-$C_{18}$alkoxy, $C_5$-$C_{12}$cycloalkoxy, $C_3$-$C_6$alkenyl, $C_7$-$C_9$phenylalkyl which is unsubstituted or mono-, di- or tri-substituted on the phenyl by $C_1$-$C_4$alkyl; or $C_1$-$C_8$acyl, $R_2$ is $C_1$-$C_{12}$alkylene, $C_2$-$C_{18}$alkylidene or one of the groups of the formulae (IIa)-(IIe)

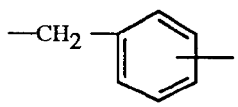 ,

(IIa)

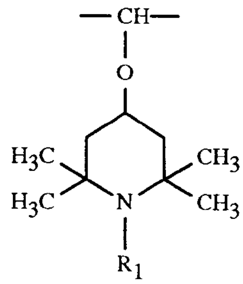 ,

(IIb)

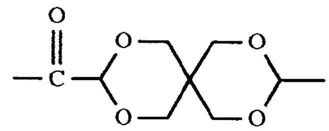 ,

(IIc)

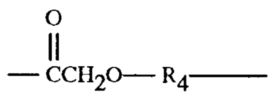 ,

(IId)

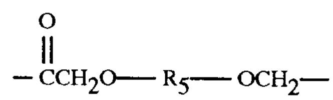

(IIe)

in which $R_1$ is as defined above, $R_4$ is methylene or phenylene and $R_5$ is $C_2$-$C_{12}$alkylene, $C_4$-$C_{12}$alkylene interrupted by 1, 2 or 3 oxygen atoms; $C_5$-$C_7$cycloalkylene, $C_5$-$C_7$cycloalkylenedi($C_1$-$C_4$alkylene), $C_2$-$C_4$alkylidenedi($C_5$-$C_7$cycloalkylene), phenylene, phenylenedi($C_1$-$C_4$alkylene), $C_1$-$C_4$alkylenediphenylene or $C_2$-$C_4$alkylidenediphenylene, where each phenylene group is unsubstituted or mono- or di-substituted by $C_1$-$C_4$alkyl; or $R_5$ is a group of the formula (IIIa) or (IIIb)

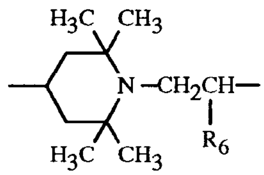 ,

(IIIa)

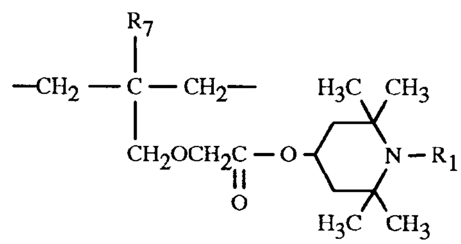

(IIIb)

in which $R_5$ is hydrogen or $C_1$-$C_4$alkyl and $R_7$ is hydrogen, $C_1$-$C_4$alkyl or a group of the formula (IV)

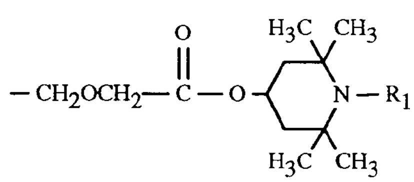 (IV).

2. A compound of the formula (I) according to claim 1, in which $R_1$ and $R_3$ which are identical or different are hydrogen, $C_1$-$C_4$alkyl, OH, $C_6$-$C_{12}$alkoxy, $C_5$-$C_8$cycloalkoxy, allyl, benzyl or acetyl.

3. A compound of the formula (I) according to claim 1, in which $R_2$ is $C_1$-$C_{10}$alkylene, $C_2$-$C_{17}$alkylidene or one of the groups of the formulae (IIa)-(IIe) in which $R_4$ is methylene or phenylene and $R_5$ is $C_2$-$C_{10}$alkylene, $C_4$-$C_{10}$alkylene interrupted by 1, 2 or 3 oxygen atoms; cyclohexylene, cyclohexylenedimethylene, isopropylidenedicyclohexylene, phenylene, xylylene, methylenediphenylene, isopropylidenediphenylene or a group of the formula (IIIa) or (IIIb) in which $R_6$ is hydrogen or methyl and $R_7$ is hydrogen, methyl, ethyl or

a group of the formula (IV).

4. A compound of the formula (I) according to claim 1, in which $R_2$ is $C_1$-$C_8$alkylene, $C_2$-$C_{15}$alkylidene or one of the groups of the formulae (IIa)-(IIe) in which $R_4$ is methylene or phenylene and $R_5$ is $C_2$-$C_8$alkylene, $C_4$-$C_8$alkylene interrupted by 1, 2 or 3 oxygen atoms; cyclohexylenedimethylene, isopropylidenedicyclohexylene, phenylene, xylylene, isopropylidenediphenylene or a group of the formula (IIIa) or (IIIb) in which $R_6$ is hydrogen or methyl and $R_7$ is methyl, ethyl or a group of the formula (IV).

5. A compound of the formula (I) according to claim 1, in which $R_2$ is $C_1$-$C_5$alkylene, $C_2$-$C_{11}$alkylidene or one of the groups of the formulae (IIa)-(IIe) in which $R_4$ is methylene and $R_5$ is $C_2$-$C_6$alkylene, $C_4$-$C_6$alkylene interrupted by 1 or 2 oxygen atoms; or a group of the formula (IIIa) or (IIIb) in which $R_6$ is hydrogen or methyl and $R_7$ is methyl or a group of the formula (IV).

6. A compound of the formula (I) according to claim 1, in which $R_1$ and $R_3$ which are identical or different are hydrogen, methyl, octoxy, cyclohexoxy, allyl or benzyl and $R_2$ is methylene or one of the groups of the formulae (IIa)-(IId) in which $R_4$ is methylene.

7. A compound according to claim 1 of the formula

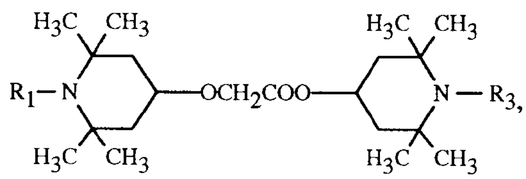

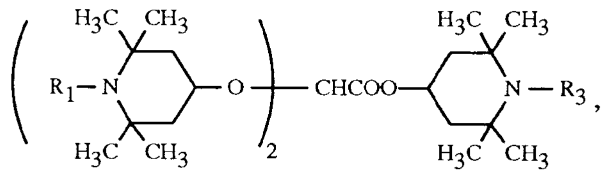

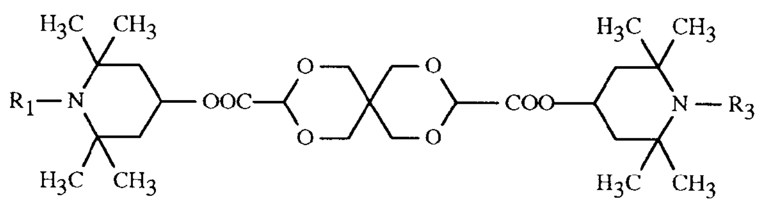

or

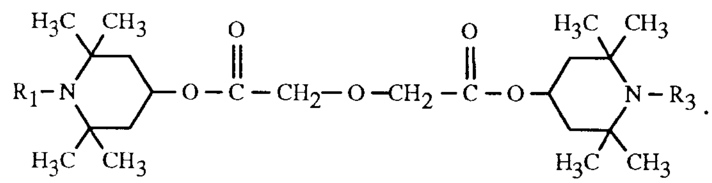

8. A composition comprising a material susceptible to degradation induced by light, heat and oxidation, and at least one compound of the formula (I) according to claim 1.

9. A composition according to claim 8, wherein the organic material is a synthetic polymer.

10. A composition according to claim 9, comprising other conventional additives for synthetic polymers in addition to the compound of the formula (I).

11. A composition according to claim 8, wherein the organic material is a polyolefin.

12. A composition according to claim 8, wherein the organic material is polyethylene or polypropylene.

13. The use of a compound of the formula (I) according to claim 1 for stabilising an organic material against degradation induced by light, heat and oxidation.

14. A process for preparing a compound of the formula (VIa)

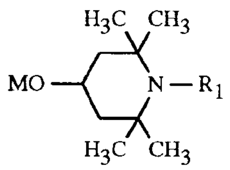

(VIa)

in which M is Na or K and $R_1$ is as defined in claim 1, comprising the reaction of a compound of the formula (VIII)

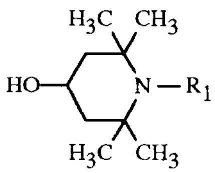

(VIII)

with NaOH or KOH in the presence of a tertiary $C_4$-$C_6$alkanol by heating under reflux and azeotropically separating off the water of reaction.

15. A process according to claim 14, wherein the reaction is carried out in the presence of a hydrocarbon solvent.

16. A process according to claim 15, wherein the solvent is hexane, cyclohexane, benzene, toluene or xylene.

17. A process according to claim 14, wherein the tertiary alkanol is 2-methyl-2-butanol.

**Claims for the following Contracting States : ES**

1. A composition comprising a material susceptible to degradation induced by light, heat and oxidation, and at least one compound of the formula (I)

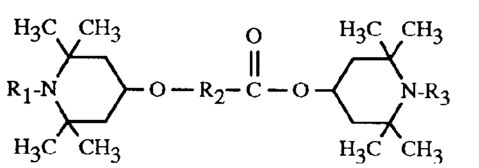

(I)

in which $R_1$ and $R_3$ which are identical or different are hydrogen, $C_1$-$C_8$alkyl, O, OH, NO, $CH_2CN$, $C_1$-$C_{18}$alkoxy, $C_5$-$C_{12}$cycloalkoxy, $C_3$-$C_6$alkenyl, $C_7$-$C_9$phenylalkyl which is unsubstituted or mono-, di- or tri-substituted on the phenyl by $C_1$-$C_4$alkyl; or $C_1$-$C_8$acyl, $R_2$ is $C_1$-$C_{12}$alkylene, $C_2$-$C_{18}$alkylidene or one of the groups of the formulae (IIa)-(IIe)

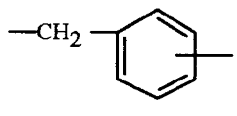

,

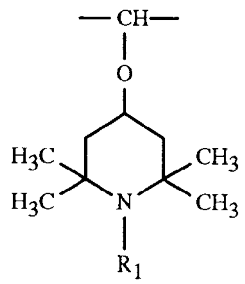

,

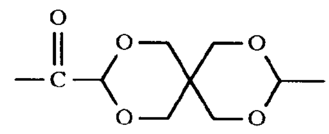

,

(IIa)

(IIb)

(IIc)

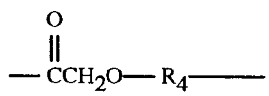

,

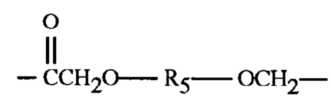

(IId)

(IIe)

in which $R_1$ is as defined above, $R_4$ is methylene or phenylene and $R_5$ is $C_2$-$C_{12}$alkylene, $C_4$-$C_{12}$alkylene interrupted by 1, 2 or 3 oxygen atoms; $C_5$-$C_7$cycloalkylene, $C_5$-$C_7$cycloalkylenedi($C_1$-$C_4$alkylene), $C_2$-$C_4$alkylidenedi($C_5$-$C_7$cycloalkylene), phenylene, phenylenedi($C_1$-$C_4$alkylene), $C_1$-$C_4$alkylenediphenylene or $C_2$-$C_4$alkylidenediphenylene, where each phenylene group is unsubstituted or mono- or di-substituted by $C_1$-$C_4$alkyl; or $R_5$ is a group of the formula (IIIa) or (IIIb)

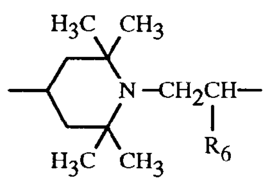

,

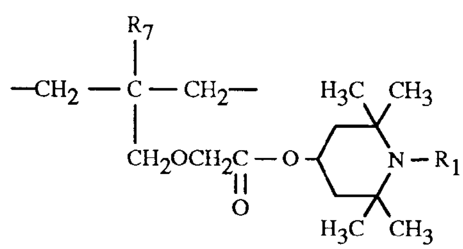

(IIIa)

(IIIb)

in which $R_5$ is hydrogen or $C_1$-$C_4$alkyl and $R_7$ is hydrogen, $C_1$-$C_4$alkyl or a group of the formula (IV) .

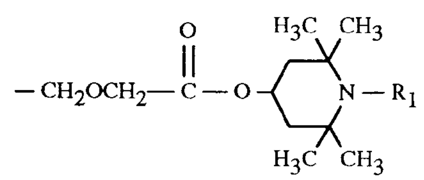

(IV) .

2. A composition according to claim 1, in which $R_1$ and $R_3$ which are identical or different are hydrogen, $C_1$-$C_4$alkyl, OH, $C_6$-$C_{12}$alkoxy, $C_5$-$C_8$cycloalkoxy, allyl, benzyl or acetyl.

3. A composition according to claim 1, in which $R_2$ is $C_1$-$C_{10}$alkylene, $C_2$-$C_{17}$alkylidene or one of the groups of the formulae (IIa)-(IIe) in which $R_4$ is methylene or phenylene and $R_5$ is $C_2$-$C_{10}$alkylene, $C_4$-$C_{10}$alkylene interrupted by 1, 2 or 3 oxygen atoms; cyclohexylene, cyclohexylenedimethylene, isopropylidenedicyclohexylene, phenylene, xylylene, methylenediphenylene, isopropylidenediphenylene or a group of the formula (IIIa) or (IIIb) in which $R_6$ is hydrogen or methyl and $R_7$ is hydrogen, methyl, ethyl or a group of the formula (IV).

4. A composition according to claim 1, in which $R_2$ is $C_1$-$C_8$alkylene, $C_2$-$C_{15}$alkylidene or one of the groups of the formulae (IIa)-(IIe) in which $R_4$ is methylene or phenylene and $R_6$ is $C_2$-$C_8$alkylene, $C_4$-$C_8$alkylene interrupted by 1, 2 or 3 oxygen atoms; cyclohexylenedimethylene, isopropylidenedicyclohexylene, phenylene, xylylene, isopropylidenediphenylene or a group of the formula (IIIa) or (IIIb) in which $R_6$ is hydrogen or methyl and $R_7$ is methyl, ethyl or a group of the formula (IV).

5. A composition according to claim 1, in which $R_2$ is $C_1$-$C_5$alkylene, $C_2$-$C_{11}$alkylidene or one of the groups of the formulae (IIa)-(IIe) in which $R_4$ is methylene and $R_5$ is $C_2$-$C_6$alkylene, $C_4$-$C_6$alkylene interrupted by 1 or 2 oxygen atoms; or a group of the formula (IIIa) or (IIIb) in which $R_6$ is hydrogen or methyl and $R_7$ is methyl or a group of the formula (IV).

6. A composition according to claim 1, in which $R_1$ and $R_3$ which are identical or different are hydrogen, methyl, octoxy, cyclohexoxy, allyl or benzyl and $R_2$ is methylene or one of the groups of the formulae (IIa)-(IId) in which $R_4$ is methylene.

7. A composition according to claim 1, in which the compound of the formula (I) is

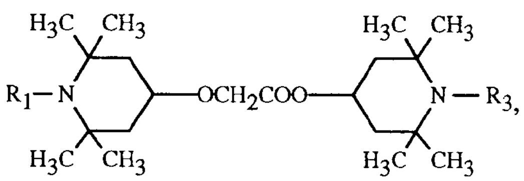

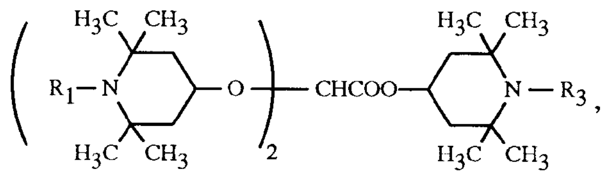

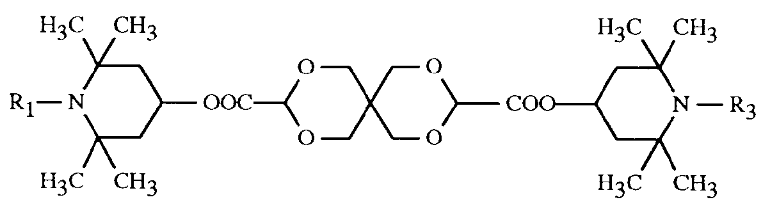

or

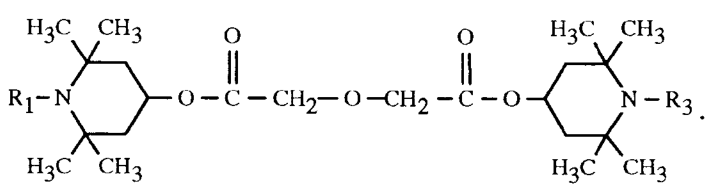

8. A composition according to claim 1, wherein the organic material is a synthetic polymer.

9. A composition according to claim 8, comprising other conventional additives for synthetic polymers in addition to the compound of the formula (I).

10. A composition according to claim 1, wherein the organic material is a polyolefin.

11. A composition according to claim 1, wherein the organic material is polyethylene or polypropylene.

**12.** A method for stabilising an organic material against degradation induced by light, heat and oxidation, which comprises incorporating into said material at least one compound of the formula (I) as defined in claim 1 .

**13.** A process for preparing a compound of the formula (I) as defined in claim 1, which comprises
1) when $R_2$ is $C_1$-$C_{12}$alkylene, $C_2$-$C_{18}$alkylidene or a group of the formula (IIa), reacting a compound of the formula (V)

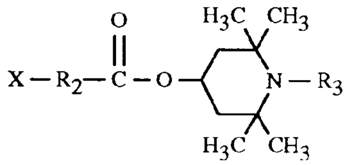
(V)

where X is Cl or Br, with a compound of the formula (VI)

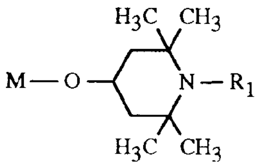
(VI)

where M is Li, Na or K;
2) when $R_2$ is a group of the formula (IIb), reacting a compound of the formula (VII)

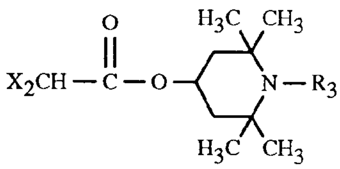
(VII)

wherein X is as defined above, with 2 mol of a compound of the formula (VI) or;
when $R_1$ = $R_3$, reacting glyoxylic acid or a $C_1$-$C_4$alkyl glyoxylate or a $C_1$-$C_4$alkyl di($C_1$-$C_4$alkoxy)acetate with 3 mol of a compound of the formula (VIII)

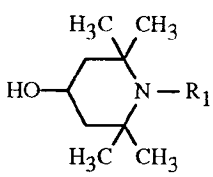
(VIII)

in the presence of an organic or inorganic acidic catalyst; or
3) when $R_2$ is a group of the formula (IIc), (IId) or (IIe), reacting 2 mol of a compound of the formula (VIII) with 1 mol of a compound of the formula (IXa), (IXb) or (IXc)

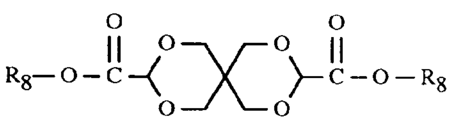 , 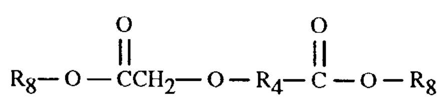 ,

(IXa) (IXb)

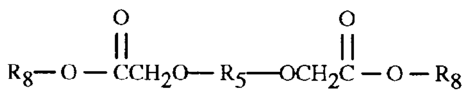

(IXc)

in which $R_8$ is $C_1$-$C_4$alkyl, in the presence of a transesterification catalyst.

**14.** A process for preparing a compound of the formula (VIa)

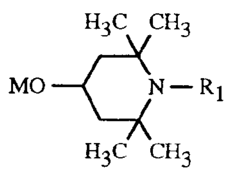

(VIa)

in which M is Na or K and $R_1$ is as defined in claim 1, comprising the reaction of a compound of the formula (VIII)

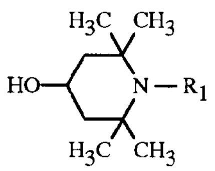

(VIII)

with NaOH or KOH in the presence of a tertiary $C_4$-$C_6$alkanol by heating under reflux and azeotropically separating off the water of reaction.

**15.** A process according to claim 14, wherein the reaction is carried out in the presence of a hydrocarbon solvent.

**16.** A process according to claim 15, wherein the solvent is hexane, cyclohexane, benzene, toluene or xylene.

**17.** A process according to claim 14, wherein the tertiary alkanol is 2-methyl-2-butanol.

European Patent Office

# EUROPEAN SEARCH REPORT

Application Number

EP 92 81 0258

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | US-A-3 984 371 (SANKYO COMPANY LIMITED) 5 October 1976 *whole document* | 1-13 | C07D211/46 C07D211/94 C07D493/10 //(C07D493/10, 319:00,319:00) C08K5/3435 |
| X | *formula I and compounds 13 and 14, column 4* --- | 1-6,8-13 | |
| Y | EP-A-0 389 420 (CIBA-GEIGY AG) 26 September 1990 *whole document* --- | 1-13 | |
| Y | EP-A-0 309 402 (CIBA-GEIGY AG) 29 March 1989 *whole document* --- | 1-13 | |
| Y | EP-A-0 153 907 (CIBA-GEIGY S.P.A.) 4 September 1985 *whole document* --- | 1-13 | |
| D,Y | US-A-4 668 722 (G.P.MACK) 26 May 1987 *whole document* --- | 1-13 | |
| Y | EP-A-0 389 419 (CIBA-GEIGY AG) 26 September 1990 *whole document* ----- | 1-13 | |
| | | | TECHNICAL FIELDS SEARCHED (Int. Cl.5 ) |
| | | | C07D C08K |

The present search report has been drawn up for all claims

| Place of search | Date of completion of the search | Examiner |
|---|---|---|
| MUNICH | 06 JULY 1992 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)